Europäisches Patentamt

European Patent Office

Office européen des brevets

(19)

(11) Veröffentlichungsnummer : **0 391 504 B1**

# (12) EUROPÄISCHE PATENTSCHRIFT

(45) Veröffentlichungstag der Patentschrift :
13.04.94 Patentblatt 94/15

(51) Int. Cl.⁵ : **C07G 17/00, A61K 35/78**

(21) Anmeldenummer : **90250010.7**

(22) Anmeldetag : **12.01.90**

(54) **Verfahren zur Extraktion von Rhizoma Petasitidis und danach herstellbares Produkt.**

(30) Priorität : **04.04.89 DE 3910831**

(43) Veröffentlichungstag der Anmeldung :
**10.10.90 Patentblatt 90/41**

(45) Bekanntmachung des Hinweises auf die
Patenterteilung :
**13.04.94 Patentblatt 94/15**

(84) Benannte Vertragsstaaten :
**AT BE CH DE DK ES FR GB GR IT LI LU NL SE**

(56) Entgegenhaltungen :
**EP-A- 0 281 656
DE-A- 1 911 862
FR-A- 8 351
PARFUMS, COSMETIQUES, AROMES, Nr. 55,
Februar-März 1984, Seiten 47-54; L. PEYRON:
"Le dioxyde de carbone liquide et supercritique, solvant d'extraction des produits naturels"**

(73) Patentinhaber : **WEBER & WEBER GmbH
Postfach 20
D-82263 Inning (DE)**

(72) Erfinder : **Koch, Volkmar, Dr.
Rosenstrasse 19
D-8084 Inning (DE)**
Erfinder : **Höcherl, Siegfried, Dipl.-Chem.
Hochstrasse 2
D-8911 Schöffelding (DE)**

(74) Vertreter : **Goddar, Heinz J., Dr. et al
FORRESTER & BOEHMERT
Franz-Joseph-Strasse 38
D-80801 München (DE)**

## Beschreibung

Die Erfindung betrifft ein Verfahren zur Extraktion von Rhizoma Petasitidis sowie ein danach herstellbares Produkt.

Die Pestwurz, Petasites hybridus oder Petasites officinalis [L.]Moendi(Astrales), wurde bereits in der Volksheilkunde wegen ihrer schleimlösenden Eigenschaften bei Husten und Asthma sowie wegen ihrer spasmolytischen Wirkung bei Krampfzuständen und Darnspasmen therapeutisch verwendet. In heutigen pharmazeutischen Präparaten kommen dabei insbesondere Extrakte von Rhizoma Petasitidis, d. h. Extrakte aus dem Wurzelstock der Pestwurz, zum Einsatz. In jüngster Vergangenheit hat sich der Indikationsbereich derartiger Extrakte noch erheblich ausgeweitet. So beschreibt beispielsweise die europäische Patentanmeldung Nr. 87 103 561.4 als zusätzliche Indikationen verschiedenartige gastrointestinale Erkrankungen.

Zur Extraktion von Rhizoma Petasitidis werden üblicherweise organische Lösungsmittel wie Methylenchlorid oder Ethanol verwendet. Dabei erhält man nach Entfernen des Lösungsmittels einen zähflüssigen Extrakt, der die lipophilere Fraktion aus der Droge enthält, daneben aber auch die im Wurzelstock der Pestwurz enthaltenen Pyrrolizidinalkaloide, die bei Verwendung der herkömmlichen Extraktionsverfahren zu etwa 40 ppm im Extrakt enthalten sind. Aufgrund des hepatotoxischen und kanzerogenen Potentials der Pyrrolizidinalkaloide ist ein solcher Gehalt in Arzneimitteln gesundheitlich nicht unbedenklich. Es gibt daher bereits intensive Bemühungen in der pharmazeutischen Industrie, den Gehalt an Pyrrolizidinalkaloiden im Extrakt von Rhizoma Petasitidis deutlich abzusenken. Alle bisherigen Versuche in dieser Richtung haben allerdings bisher noch nicht zu einem wesentlichen Erfolg geführt.

Der Erfindung liegt daher die Aufgabe zugrunde, ein gattungsgemäßes Verfahren zu schaffen, mit dem es möglich ist, den Gehalt an Pyrrolizidinalkaloiden im Extrakt von Rhizoma Petasitidis deutlich abzusenken, vorzugsweise bis unterhalb der Nachweisgrenze von 0.1 ppm.

Erfindungsgemäß wird diese Aufgabe bei einem gattungsgemäßen Verfahren dadurch gelöst, daß als Extraktionsmittel gasförmiges Kohlendioxid in überkritischem Zustand verwendet wird.

Dabei wird bevorzugt mit einem Kohlendioxiddruck von zwischen 100 und 400 bar, besonders bevorzugt etwa 250 bar gearbeitet.

Das erfindungsgemäße Verfahren kann besonders effektiv durchgeführt werden, wenn der Wassergehalt der zu extrahierenden Droge oberhalb von 5 Gew.-%, bevorzugt bei etwa 10 Gew.-% liegt.

Weitere vorteilhafte Bedingungen für die Durchführung des erfindungsgemäßen Verfahrens sind:

| | |
|---|---|
| Kohlendioxiddurchsatz | 200 - 500 kg/h, bevorzugt etwa 270 kg/h; |
| Extraktionsdauer | 100 bis 200 min, bevorzugt 150 min; |
| Extraktionstemperatur | etwa 35°C. |

Gegenstand der Erfindung ist weiterhin ein nach dem erfindungsgemäßen Verfahren herstellbarer Extrakt aus Rhizoma Petasitidis mit einem Gehalt an Pyrrolizidinalkaloiden von unter 5 ppm, wobei auch ein Absenken des Gehaltes auf unter die Nachweisgrenze von 0,1 ppm bei geeigneter Wahl der Verfahrensbedingungen erreichbar ist.

Das erfindungsgemäß angewendete Verfahren ist als solches bereits bekannt und wurde vor etwa 10 Jahren von K. Zosel entwickelt. Diese sogenannte Destraktion beruht auf der Erkenntnis, daß bei gewissen Gasen im überkritischen Zustand ein sprunghafter Anstieg des Lösungsvermögens für bestimmte Stoffe auftritt. Die Destraktion wurde bisher hauptsächlich zur Extraktion von Naturstoffen (z. B. zur Entcoffeinierung von Kaffee), von Kohlenwasserstoffen (z. B. des Erdöls), in der Gaschromatographie und bei vielen anderen Stofftrennungen eingesetzt.

In völlig überraschender Weise führt dieses Verfahren auch zur Lösung der dieser Erfindung zugrundeliegenden Aufgabe. Von der Theorie her wäre zu erwarten gewesen, daß bei Verwendung von überkritischem Kohlendioxid als Extraktionsmittel durch dessen hervorragende Lösungseigenschaften ein Anstieg des Gehalts an Pyrrolizidinalkaloiden im Extrakt gegenüber der eingesetzten Droge zu beobachten wäre. Wurden die Alkaloide quantitativ extrahiert, müßten sie im Extrakt um das 50-fache angereichert werden. Tatsächlich und völlig überraschend hat sich aber ergeben, daß bei Auswahl geeigneter Verfahrensbedingungen ganz im Gegensatz zur Erwartung ein Absenken des Gehaltes an Pyrrolizidinalkaloiden im Extrakt auf unter die Nachweisgrenze von 0,1 ppm möglich ist.

Weitere Vorteile und Eigenschaften der Erfindung ergeben sich aus den nachfolgenden Beispielen.

Beispiele 1 bis 4

In jedem Versuch wurde eine Charge Rhizoma Petasitidis zu je 60 kg nach dem üblichen Destraktionsverfahren mit Kohlendioxid extrahiert. Dabei wurden als Verfahrensparameter der Wassergehalt, der Kohlendioxiddruck, der Kohlendioxiddurchsatz und die Extraktionsdauer variiert. Ein Wassergehalt von um die 10 Gew.-% liegt dabei bei der verwendeten Droge im Normbereich. Bei zwei Chargen wurde eine nachgetrocknete Droge verwendet, bei der der Wassergehalt auf weniger als 5 % abgesenkt worden war. Die Ausbeute der Extraktion betrug in jedem Falle etwa 2 %.

Die Versuchsergebnisse, die in der nachfolgenden Tabelle zusammengestellt sind, lassen erkennen, daß insbesondere die Anwendung eine hohen Kohlendioxiddrucks bei einer nicht nachgetrockneten Droge zum deutlich besten Ergebnis führt, wobei aber auch die in den anderen Versuchen erzielten Gehalte an Pyrrolizidinalkaloiden im Extrakt um mehr als eine Zehnerpotenz unter den bisher erreichbaren Werten liegen.

Die in der vorstehenden Beschreibung sowie den Ansprüchen offenbarten Merkmale der Erfindung können sowohl einzeln als auch in beliebiger Kombination für die Verwirklichung der Erfindung in ihren verschiedenen Ausführungsformen wesentlich sein.

TABELLE

| Versuch: | Wassergehalt | $CO_2$-Druck | $CO_2$-Durchsatz | Temperatur | Extraktionsdauer | Pyrrolizidinalkaloidgehalt des Extrakts |
|---|---|---|---|---|---|---|
| 1 | etwa 3 % | 250bar | 270kg/h | 35°C | 155 min | 3,2ppm |
| 2 | etwa 9 % | 250bar | 270kg/h | 35°C | 155 min | nicht nachweisbar bei einer Nachweisgrenze von 0,1 µg/g (= 0,1ppm) |
| 3 | ≤ 5 % | 130bar | 345kg/h | 35°C | 120 min | 2,4ppm |
| 4 | etwa 9 % | 130bar | 345kg/h | 35°C | 120 min | 1,6ppm |

EP 0 391 504 B1

**Patentansprüche**

**Patentansprüche für folgende Vertragsstaaten : DE, GB, FR, IT, NL, SE, LI, CH, BE, AT, LU, DK**

1. Verfahren zur Extraktion von Rhizoma Petasitidis, dadurch gekennzeichnet, daß als Extraktionsmittel gasförmiges Kohlendioxid in überkritischem Zustand verwendet wird.

2. Verfahren nach Anspruch 1, dadurch gekennzeichnet, daß mit einem Kohlendioxiddruck von zwischen 100 und 400 bar gearbeitet wird.

3. Verfahren nach Anspruch 2, dadurch gekennzeichnet, daß der Kohlendioxiddruck bei etwa 250 bar liegt.

4. Verfahren nach einem der vorhergehenden Ansprüche, dadurch gekennzeichnet, daß mit einem Kohlendioxiddurchsatz von zwischen 200 und 500 kg/h gearbeitet wird.

5. Verfahren nach Anspruch 4, dadurch gekennzeichnet, daß der Kohlendioxiddurchsatz bei etwa 270 kg/h liegt.

6. Verfahren nach einem der vorhergehenden Ansprüche, dadurch gekennzeichnet, daß die Dauer der Extraktion zwischen 100 und 200 min liegt.

7. Verfahren nach Anspruch 6, dadurch gekennzeichnet, daß die Dauer der Extraktion bei etwa 150 min liegt.

8. Verfahren nach einem der vorhergehenden Ansprüche, dadurch gekennzeichnet, daß die Extraktionstemperatur bei etwa 35°C liegt.

9. Verfahren nach einem der vorangehenden Ansprüche, dadurch gekennzeichnet, daß der Wassergehalt der zu extrahierenden Droge oberhalb von 5 Gew.-% liegt.

10. Verfahren nach Anspruch 9, dadurch gekennzeichnet, daß der Wassergehalt bei etwa 10 Gew.-% liegt.

11. Extrakt aus Rhizoma Petasitidis mit einem Gehalt an Pyrrolizidinalkaloiden von unter 5 ppm, herstellbar mit einem Verfahren nach einem der vorangehenden Ansprüche.

12. Extrakt nach Anspruch 11, mit einem Gehalt an Pyrrolizidinalkaloiden von unter 0,1 ppm.

**Patentansprüche für folgende Vertragsstaaten: GR, ES**

1. Verfahren zur Extraktion von Rhizoma Petasitidis, dadurch gekennzeichnet, daß als Extraktionsmittel gasförmiges Kohlendioxid in überkritischem Zustand verwendet wird.

2. Verfahren nach Anspruch 1, dadurch gekennzeichnet, daß mit einem Kohlendioxiddruck von zwischen 100 und 400 bar gearbeitet wird.

3. Verfahren nach Anspruch 2, dadurch gekennzeichnet, daß der Kohlendioxiddruck bei etwa 250 bar liegt.

4. Verfahren nach einem der vorhergehenden Ansprüche, dadurch gekennzeichnet, daß mit einem Kohlendioxiddurchsatz von zwischen 200 und 500 kg/h gearbeitet wird.

5. Verfahren nach Anspruch 4, dadurch gekennzeichnet, daß der Kohlendioxiddurchsatz bei etwa 270 kg/h liegt.

6. Verfahren nach einem der vorhergehenden Ansprüche, dadurch gekennzeichnet, daß die Dauer der Extraktion zwischen 100 und 200 min liegt.

7. Verfahren nach Anspruch 6, dadurch gekennzeichnet, daß die Dauer der Extraktion bei etwa 150 min liegt.

8. Verfahren nach einem der vorhergehenden Ansprüche, dadurch gekennzeichnet, daß die Extraktionstemperatur bei etwa 35°C liegt.

**9.** Verfahren nach einem der vorangehenden Ansprüche, dadurch gekennzeichnet, daß der Wassergehalt der zu extrahierenden Droge oberhalb von 5 Gew.-% liegt.

**10.** Verfahren nach Anspruch 9, dadurch gekennzeichnet, daß der Wassergehalt bei etwa 10 Gew.-% liegt.

**Claims**

**Claims for the following Contracting States : DE, GB, FR, IT, NL, SE, LI, CH, BE, AT, LU, DK**

**1.** A process for the extraction of Rhizoma petasitidis, characterised in that the extraction medium used is gaseous carbon dioxide in the super-critical state.

**2.** A process according to claim 1, characterised in that a carbon dioxide pressure of between 100 and 400 bar is used.

**3.** A process according to claim 2, characterised in that the carbon dioxide pressure is about 250 bar.

**4.** A process according to any one of the preceding claims, characterised in that a carbon dioxide throughput of between 200 and 500 kg/h is used.

**5.** A process according to claim 4, characterised in that the carbon dioxide throughput is about 270 kg/h.

**6.** A process according to any one of the preceding claims, characterised in that the duration of the extraction is between 100 and 200 minutes.

**7.** A process according to claim 6, characterised in that the duration of the extraction is about 150 minutes.

**8.** A process according to any one of the preceding claims, characterised in that the extraction temperature is about 35°C.

**9.** A process according to any one of the preceding claims, characterised in that the water content of the drug for extraction is above 5% by weight.

**10.** A process according to claim 9, characterised in that the water content is about 10% by weight.

**11.** An extract of Rhizoma petasitidis having a pyrrolizidine alkaloid content of less than 5 ppm, adapted to be prepared by a process according to any one of the preceding claims.

**12.** An extract according to claim 11, having a pyrrolizidine alkaloid content of less than 0.1 ppm.

**Claims for the following Contracting States : GR, ES**

**1.** A process for the extraction of Rhizoma petasitidis, characterised in that the extraction medium used is gaseous carbon dioxide in the super-critical state.

**2.** A process according to claim 1, characterised in that a carbon dioxide pressure of between 100 and 400 bar is used.

**3.** A process according to claim 2, characterised in that the carbon dioxide pressure is about 250 bar.

**4.** A process according to any one of the preceding claims, characterised in that a carbon dioxide throughput of between 200 and 500 kg/h is used.

**5.** A process according to claim 4, characterised in that the carbon dioxide throughput is about 270 kg/h.

**6.** A process according to any one of the preceding claims, characterised in that the duration of the extraction is between 100 and 200 minutes.

**7.** A process according to claim 6, characterised in that the duration of the extraction is about 150 minutes.

8. A process according to any one of the preceding claims, characterised in that the extraction temperature is about 35°C.

9. A process according to any one of the preceding claims, characterised in that the water content of the drug for extraction is above 5% by weight.

10. A process according to claim 9, characterised in that the water content is about 10% by weight.

## Revendications

**Revendications pour les Etats contractants suivants : DE, GB, FR, IT, NL, SE, LI, AT, LU, DK, CH, BE**

1. Procédé pour l'extraction de Rhizoma Petasitidis, caractérisé en ce que l'on utilise en tant qu'agent d'extraction du dioxyde de carbone gazeux à l'état surcritique.

2. Procédé selon la revendication 1, caractérisé en ce que l'on opère avec une pression de dioxyde de carbone de 100 à 400 bar.

3. Procédé selon la revendication 2, caractérisé en ce que la pression de dioxyde de carbone est d'environ 250 bar.

4. Procédé selon une des revendications qui précèdent, caractérisé en ce que l'on opère avec un débit de dioxyde de carbone de 200 à 500 kg/h.

5. Procédé selon une des revendications qui précèdent, caractérisé en ce que le débit de dioxyde de carbone est d'environ 270 kg/h.

6. Procédé selon une des revendications qui précèdent, caractérisé en ce que la durée de l'extraction est de 100 à 200 min.

7. Procédé selon la revendication 6, caractérisé en ce que la durée d'extraction est d'environ 150 min.

8. Procédé selon une des revendications qui précèdent, caractérisé en ce que la température d'extraction est d'environ 35°C.

9. Procédé selon une des revendications qui précèdent, caractérisé en ce que la teneur en humidité de la drogue à extraire est supérieure à 5 % en poids.

10. Procédé selon la revendication 9, caractérisé en ce que la teneur en humidité est d'environ 10 % en poids.

11. Extrait de Rhizoma Petasitidis contenant moins de 5 ppm d'alcaloides pyrrolizidiniques, préparé par un procédé selon une des revendications qui précèdent.

12. Extrait selon la revendication 11, dont la teneur en alcaloides pyrrolizidiniques est inférieure à 0,1 ppm.

**Revendications pour les Etats contractants suivants : ES, GR**

1. Procédé pour l'extraction de Rhizoma Petasitidis, caractérisé en ce que l'on utilise en tant qu'agent d'extraction du dioxyde de carbone gazeux à l'état surcritique.

2. Procédé selon la revendication 1, caractérisé en ce que l'on opère avec une pression de dioxyde de carbone de 100 à 400 bar.

3. Procédé selon la revendication 2, caractérisé en ce que la pression de dioxyde de carbone est d'environ 250 bar.

4. Procédé selon une des revendications qui précèdent, caractérisé en ce que l'on opère avec un débit de dioxyde de carbone de 200 à 500 kg/h.

5. Procédé selon la revendication 4, caractérisé en ce que le débit de dioxyde de carbone est d'environ 270 kg/h.

6. Procédé selon une des revendications qui précèdent, caractérisé en ce que la durée d'extraction est de 100 à 200 min.

7. Procédé selon la revendication 6, caractérisé en ce que la durée de l'extraction est d'environ 150 min.

8. Procédé selon une des revendications qui précèdent, caractérisé en ce que la température d'extraction est d'environ 35°C.

9. Procédé selon une des revendications qui précèdent, caractérisé en ce que la teneur en humidité de la drogue à extraire est supérieure à 5 % en poids.

10. Procédé selon la revendication 9, caractérisé en ce que la teneur en humidité est d'environ 10 % en poids.